# EUROPEAN PATENT SPECIFICATION

(11) **EP 4 166 183 B1**
(45) Date of publication and mention of the grant of the patent: **03.09.2025**
(21) Application number: 21842549.4
(22) Date of filing: 16.07.2021
(51) Int. Cl.: A61M 16/16

(54) **HUMIDIFICATION TANK AND VENTILATION TREATMENT DEVICE**
BEFEUCHTUNGSTANK UND BELÜFTUNGSBEHANDLUNGSVORRICHTUNG
RÉSERVOIR D'HUMIDIFICATION ET DISPOSITIF DE TRAITEMENT DE VENTILATION

(30) Priority: 17.07.2020 CN 202021424983 U; 17.07.2020 CN 202021420609 U; 17.07.2020 CN 202021425631 U; 17.07.2020 CN 202021420610 U
(43) Date of publication of application: 19.04.2023
(73) Proprietor: BMC (Tianjin) Medical Co., Ltd., Tianjin 301700 (CN)
(72) Inventor: TIAN, Xin, Tianjin 301700 (CN); CHEN, Yunjing, Tianjin 301700 (CN); LIU, Jiuzhong, Tianjin 301700 (CN); ZHUANG, Zhi, Beijing 100041 (CN)
(74) Representative: Grünecker Patent- und Rechtsanwälte PartG mbB
(86) International application number: PCT/CN2021/106880
(87) International publication number: WO 2022/012679

(56) References cited:
- WO-A1-2007/019626
- WO-A1-2018/040975
- CN-A- 104 602 743
- CN-A- 111 408 008
- CN-U- 201 668 840
- CN-U- 201 668 840
- CN-U- 204 563 241
- CN-U- 207 119 048
- CN-U- 207 694 060
- CN-U- 209 347 875
- US-A- 3 265 067
- US-A1- 2013 008 440
- US-A1- 2016 129 212

## Description

### TECHNICAL FIELD

The present application relates to the field of respiratory devices and, more particularly, to a humidification tank, and relates to a ventilation treatment device.

### BACKGROUND

A ventilator is a necessary auxiliary device for maintaining respiration and ensuring ventilation of a respiratory failure patient, which conveys mixed air and oxygen to an airway of the patient during assisted respiration. Usually, oxygen provided by a wall-mounted oxygen setup or an oxygen cylinder is in a dry and cold state, so it is necessary to heat and humidify the mixed gas through a humidification tank to fully humidify a respiratory tract and protect trachea, bronchi and mucous membranes. Nowadays, in order to reduce the cost of using disposable medical consumables and the risk of treating medical wastes, it is necessary to design a humidification tank that can be used repeatedly disinfected and used.

In an existing humidification tank, a gas humidifying path is too short, and an airflow is basically unidirectional. After the gas enters a tank body, the gas flows at a high speed, the direction of the airflow is chaotic, and a humidifying time of the gas is too short, so it is difficult to achieve an ideal humidifying effect.

CN 201668840 U discloses a medical self-heating humidifying bottle, wherein an inverted Y-shaped intake pipe is arranged outside the heating agent tube in the humidifying bottle. CN 207694060 U discloses a medical breathing machine humidifier, wherein a plurality of horizontal air intake branches are equipped at the bottom side of an intake pipe. WO 2018/040975 A1 discloses an air-guiding tube component, a liquid-storage device, a humidifier, and a ventilator, wherein the air-guiding tube component comprises a guiding tube having a communication port communicating with the air-inlet end of the air-outlet guiding tube, and a plurality of air-inlet end of the air-outlet guiding tube, and a plurality of air-reflux ends communicating with the communication port.

### SUMMARY

The present application aims at providing a humidification tank to solve a problem of single airflow direction in a tank body.

The invention is defined in the appended independent claim 1. Preferred embodiments are matter of the dependent claims.

To implement the foregoing object, a first aspect of the present application provides a humidification tank, wherein the humidification tank includes a tank body provided with a cavity, a joint pipe inserted into the cavity and at least two branch pipes communicated with an outlet end of the joint pipe, wherein the cavity is provided with two deflection plates each facing one of outlet ends of the branch pipes, and the deflection plates are configured to change a direction of gas flowing out of the outlet ends of the branch pipes, and ends of the two deflection plates close to each other are spaced to form a flow channel.

Further, the outlet end of the joint pipe is connected to the two branch pipes which extend horizontally, and the two branch pipes and the joint pipe form a Y-shaped three-way pipe.

Further, ends of the two deflection plates far away from each other are both connected to a side wall of the tank body.

Further, the deflection plate is connected to a hanging wall of the tank body, and the deflection plate is spaced from a bottom wall of the tank body.

Further, the deflection plate is recessed towards the outlet end of the branch pipe.

Further, the cavity is provided with a baffle facing the flow channel, and the baffle is configured to change the direction of the gas flowing out of the outlet of the branch pipe.

Further, two lateral sides of the baffle are spaced from the side wall of the tank body.

Further, the baffle is connected to the hanging wall of the tank body, and the baffle is spaced from the bottom wall of the tank body.

Further, the humidification tank includes an exhaust pipe connected to the hanging wall of the tank body, and the baffle is located between the flow channel and an inlet end of the exhaust pipe.

Further, the hanging wall of the tank body is connected to a flow guide plate, the inlet end of the exhaust pipe is located between the baffle and the flow guide plate, and a lower end of the flow guide plate is inclined towards the baffle.

Further, the baffle is recessed towards the exhaust pipe.

To implement the foregoing object, a second aspect of the present application provides a humidification tank, wherein the humidification tank includes a tank body provided with a cavity and a joint pipe inserted into the cavity, and a part of the joint pipe located in the cavity is provided with a plurality of air holes at intervals along a circumferential direction.

Further, the plurality of air holes are distributed in an array along circumferential and axial directions of the joint pipe.

Further, the tank body includes a hanging wall, and the joint pipe extends horizontally and is arranged at intervals from the hanging wall.

Further, the hanging wall includes an arc-shaped portion covering the joint pipe.

Further, the cavity is provided with a baffle spaced from an outlet end of the joint pipe.

Further, the humidification tank includes an exhaust pipe connected to the hanging wall, and the baffle is located between the joint pipe and the exhaust pipe.

Further, the baffle is recessed towards the joint pipe.

Further, one side edge of an inlet of the exhaust pipe far away from the baffle is connected to a flow guide plate, and a lower end of the flow guide plate is inclined toward the baffle.

Further, an opening of the joint pipe located in the cavity is provided with a sealing wall.

Further, the tank body includes a side wall, and the joint pipe passes through the side wall and is inserted into the cavity of the tank body.

Further, an outer peripheral surface of the joint pipe is provided with a buckle, and the side wall is provided with a clamping groove which can be clamped and matched with the buckle.

Further, the outer circumferential surface of the joint pipe is provided with a guide rib, and the side wall is provided with a guide groove which can accommodate insertion of the guide rib.

To implement the foregoing object, a third aspect of the present application provides a humidification tank, wherein the humidification tank includes a tank body provided with a cavity, a joint pipe inserted into the cavity and a baffle located in the cavity and spaced from an outlet end of the joint pipe, and the baffle is recessed towards the joint pipe.

Further, the tank body includes a hanging wall, and the baffle is connected to the hanging wall.

Further, the humidification tank includes an exhaust pipe connected to the hanging wall, and the baffle is located between an inlet end of the exhaust pipe and an outlet end of the joint pipe.

Further, one side edge of an inlet of the exhaust pipe far away from the baffle is connected to a flow guide plate, and a lower end of the flow guide plate is inclined toward the baffle.

Further, a middle portion of the baffle facing the joint pipe is recessed relative to two lateral sides of the baffle.

Further, the tank body includes a side wall, and the joint pipe passes through the side wall and is inserted into the cavity of the tank body.

Further, an outer peripheral surface of the joint pipe is provided with a buckle, and the side wall is provided with a clamping groove which can be clamped and matched with the buckle.

Further, the outer circumferential surface of the joint pipe is provided with a guide rib, and the side wall is provided with a guide groove which can accommodate insertion of the guide rib.

To implement the foregoing object, a fourth aspect of the present application provides a humidification tank, wherein the humidification tank includes a tank body provided with a cavity, a joint pipe inserted into the cavity and a baffle, wherein the baffle extends from an A side to a B side in a lateral direction of an outlet end of the joint pipe, and the baffle deflects away from the joint pipe.

Further, a side edge of the baffle located at the B side is connected to a side wall of the tank body.

Further, an opening is formed at the B side of the outlet end of the joint pipe.

Further, the outlet end of the joint pipe is connected to an elbow bent from the A side to the B side.

Further, a hanging wall of the tank body is connected to an exhaust pipe, and the baffle is located between the joint pipe and an inlet end of the exhaust pipe.

Further, the baffle is connected to the hanging wall of the tank body, and is spaced from a bottom wall of the tank body.

Further, one side edge of an inlet of the exhaust pipe close to the baffle is connected to a flow guide plate, and a lower end of the flow guide plate is inclined towards the baffle.

Further, the joint pipe horizontally extends through the side wall of the tank body.

Further, an outer periphery of the joint pipe is provided with a guide rib, and the side wall is provided with a guide groove which can accommodate insertion of the guide rib.

Further, an outer peripheral surface of the joint pipe is provided with a buckle, and the side wall is provided with a clamping groove which can be clamped and matched with the buckle.

Moreover, the present application further provides a ventilation treatment device, wherein the ventilation treatment device includes the humidification tank described in the above solutions.

By means of the foregoing technical solution, the branch pipes can provide airflows in different directions, so that the gas reaches more positions in the cavity, a situation that positions which cannot be blown by the gas exist in the cavity can be avoided, airflow dead zones are avoided, gas flowability in the cavity is improved, and a gas flowing path is prolonged, so that a humidifying effect of the gas is improved.

### BRIEF DESCRIPTION OF THE DRAWINGS

FIG. 1 is a perspective diagram of a humidification tank according to the embodiments of the present application;
FIG. 2 is a schematic structural diagram of the joint pipe and the branch pipe according to the embodiments of the present application;
FIG. 3 is a sectional view along the line A-A in FIG. 2;
FIG. 4 is a sectional view of the humidification tank according to the embodiments of the present application;
FIGs. 5- 8 are schematic diagrams showing internal structures of the humidification tank according to the embodiments of the present application;
FIG. 9 is a perspective diagram of the humidification tank according to the embodiments of the present application;
FIG. 10 is an internal structure diagram of the humidification tank according to the embodiments of the present application viewed from the bottom;
FIG. 11 is a sectional view along the line A-A in FIG. 10;
FIG. 12 is a sectional view along the line B-B in FIG. 10;
FIG. 13 is a schematic structural diagram of the joint pipe according to the embodiments of the present application;
FIG. 14 is a perspective diagram of the humidification tank according to the embodiments of the present application;
FIG. 15 is an internal structure diagram of the humidification tank according to the embodiments of the present application viewed from the bottom;
FIG. 16 is a sectional view along the line A-A in FIG. 15;
FIG. 17 is a perspective diagram of the humidification tank according to the embodiments of the present application;
FIG. 18 is a sectional view of the humidification tank according to the embodiments of the present application;
FIG. 19 and FIG. 20 are schematic diagrams showing internal structures of the humidification tank according to the embodiments of the present application;
FIG. 21 is a schematic structural diagram of the joint pipe according to the embodiments of the present application;
FIG. 22 is a perspective diagram of the humidification tank according to the embodiments of the present application;
FIG. 23 is a schematic diagram showing an internal structure of the humidification tank according to the embodiments of the present application;
FIG. 24 is a schematic structural diagram of the joint pipe according to the embodiments of the present application;
FIG. 25 is a perspective diagram of the joint pipe according to the embodiments of the present application;
FIG. 26 is a perspective view of the humidification tank according to the embodiments of the present application;
FIG. 27 is a schematic diagram showing an internal structure of the humidification tank according to the embodiments of the present application; and
FIG. 28 is a perspective diagram of the joint pipe and the elbow according to the embodiments of the present application.

Description of Reference Numerals
100 refers to tank body, 101 refers to hanging wall, 102 refers to side wall, 103 refers to bottom ball, 104 refers to baffle, 105 refers to exhaust pipe, 106 refers to flow guide plate, 107 refers to clamping groove, 108 refers to guide groove, 109 refers to deflection plate, 200 refers to joint pipe, 201 refers to buckle, 202 refers to guide rib, 203 refers to air hole, 300 refers to branch pipe, and 400 refers to elbow.

### DETAILED DESCRIPTION OF THE EMBODIMENTS

The embodiments of the present application will be further described hereinafter in detail with reference to the drawings. It should be understood that the specific embodiments described herein are only for the purpose of illustration and explanation of the present application and are not intended to limit the present application.

### Embodiment 1

The present application provides a humidification tank, wherein the humidification tank includes a tank body provided with a cavity 100, a joint pipe 200 inserted into the cavity and at least two branch pipes 300 communicated with an outlet end of the joint pipe 200.

The tank body 100 is provided with the cavity for containing liquid, such as containing water. The joint pipe 200 is inserted into the cavity of the tank body 100, and one end of the joint pipe located outside the cavity may be connected to an air guide pipe, so that gas can be input into the cavity. The branch pipes 300 extend from the joint pipe 200 in different directions, and can blow airflows from the joint pipe 200 in different directions into the cavity, so that the gas reaches more positions in the cavity, a situation that positions which cannot be blown by the gas exist in the cavity can be avoided, airflow dead zones are avoided, gas flowability in the cavity is improved, and a gas flowing path is prolonged, so that a humidifying effect of the gas is improved.

The tank body 100 is connected to an exhaust pipe 105, the exhaust pipe 105 can exhaust the humidified gas, and the exhaust pipe 105 may be connected to the air guide pipe to output the gas to a target position.

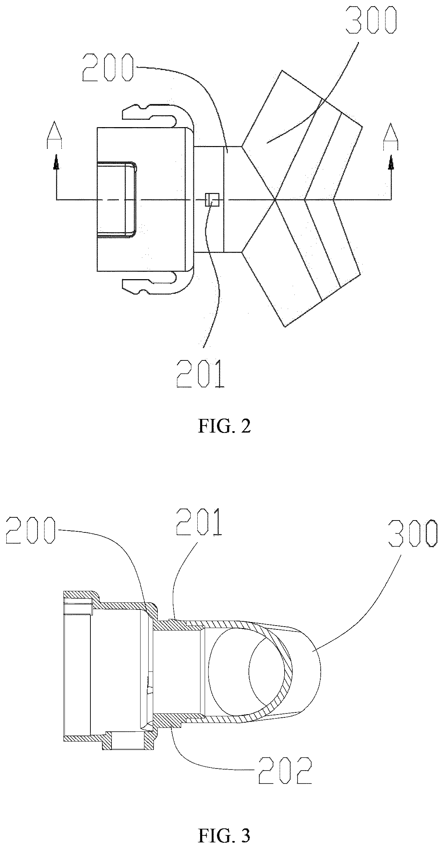

Further, the joint pipe 200 may pass through a side wall 102 of the tank body 100, the outlet end of the joint pipe 200 is connected to two branch pipes which extend horizontally, and the two branch pipes 300 and the joint pipe 200 form a Y-shaped three-way pipe. Referring to FIGs. 2 to 8, only two branch pipes 300 are connected to the joint pipe 200, and the branch pipes 300 are located at the outlet end of the joint pipe 200. The joint pipe 200 and the branch pipes 300 are substantially formed into the Y-shaped three-way pipe, and the two branch pipes 300 face each other to form an included angle, which can make the airflow be conveyed to both sides and reach more positions in comparison to that of only one joint pipe 200. Certainly, in other embodiments, the joint pipe 200 may be connected to the branch pipe 300 at a position other than the outlet end, and the branch pipe 300 may extend obliquely upward or downward.

Further, the cavity is provided with a deflection plate 109 facing an outlet end of the branch pipe 300, and the deflection plate 109 is configured to change a direction of gas flowing out of an outlet of the branch pipe 300. The gas discharged from the branch pipe 300 may be blown into the deflection plate 109, and the deflection plate 109 can guide to change an original direction of the airflow, so that the airflow can be dispersed into more directions, and can be further conveyed to more positions in the cavity, so that the airflow circulation in the cavity can be strengthened and dead zones can be avoided.

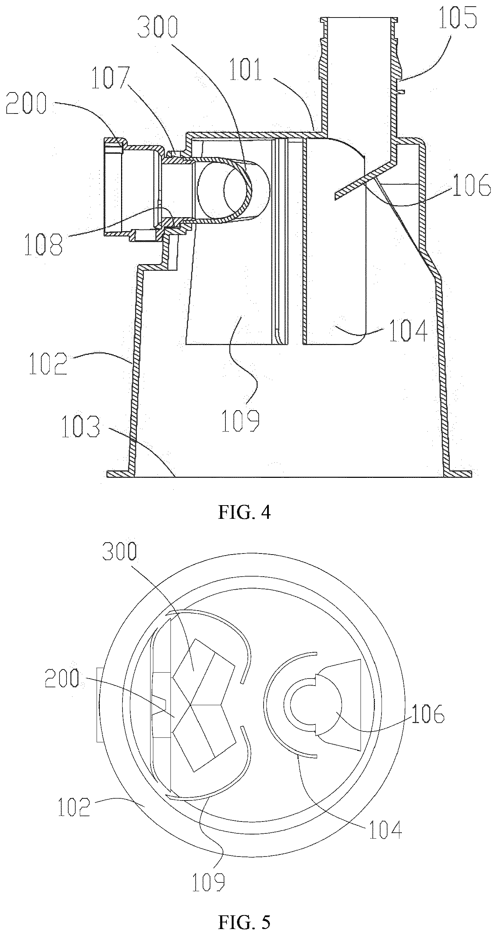

Ends of the two deflection plates 109 close to each other are spaced to form a flow channel. As shown in FIG. 5 to FIG. 8, the deflection plates 109 are spaced to form the flow channel, which allows the airflows discharged from the two branch pipes 300 to pass through the flow channel.

Moreover, ends of the two deflection plates 109 far away from each other are both connected to the side wall 102 of the tank body 100. The two deflection plates 109 and a part of the side wall 102 enclose a space provided with a channel outlet, and the airflow from the branch pipe 300 circulates in this space and is discharged from the channel. In this structure, the airflow can reach the side wall 102 near the joint pipe 200, avoiding the formation of a dead zone here.

Moreover, the deflection plate 109 is connected to a hanging wall 101 of the tank body 100, and the deflection plate 109 is spaced from a bottom wall 103 of the tank body 100. The airflow blown from the branch pipe 300 to the deflection plate 109 can cross a lower edge of the deflection plate 109 after changing direction to reach the other side of the deflection plate 109.

Alternatively, the deflection plate 109 is recessed towards the branch pipe 300. The branch pipe faces a middle portion of the deflection plate 109, and the airflow discharged from the branch pipe 300 changes the direction after reaching a middle portion of a surface of the deflection plate 109, so that the airflow flows reversely under the guidance of two lateral sides.

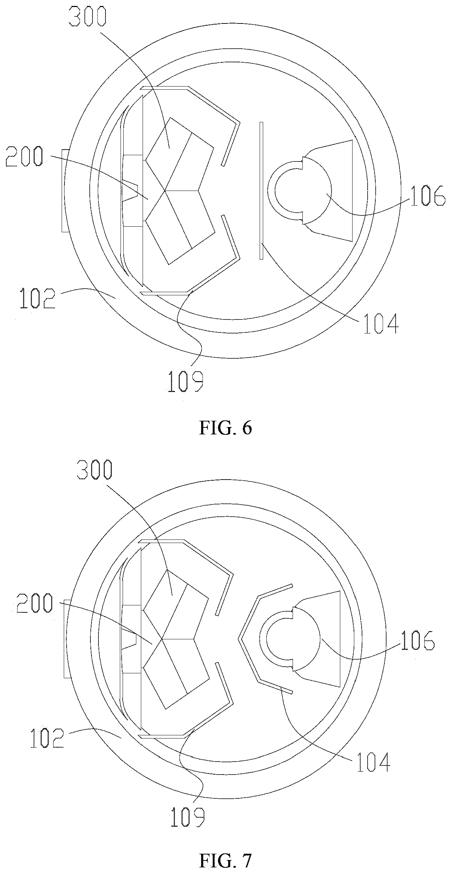

With reference to FIG. 5, the deflection plate 109 is substantially formed into a concave arc, and as shown in FIGs. 6 to 8, the deflection plate 109 includes a section of flat plate in the middle and a section of flat plate on both sides, and the flat plates on both sides are inclined relative to the flat plate in the middle.

Moreover, the cavity is provided with a baffle 104 facing the flow channel, and the baffle 104 is configured to change the direction of the gas flowing out of the outlet of the branch pipe 300. The airflow discharged from the flow channel can be blown onto the baffle 104, and the direction of the airflow can be further changed by the baffle 104.

Two lateral sides of the baffle 104 are spaced from the side wall 102 of the tank body 100. The airflow blown from the flow channel to the baffle 104 can cross the baffle 104 from the two lateral edges of the baffle 104 and reach the other side of the baffle 104.

The baffle 104 is connected to the hanging wall 101 of the tank body 100, and the baffle 104 is spaced from the bottom wall 103 of the tank body 100. The airflow blown from the flow channel to the baffle 104 may pass over the baffle 104 from a lower side of the baffle 104, so that the airflow may be closer to the bottom wall 103 of the tank body 100, i.e. closer to the water at a bottom portion of the tank body 100, thereby achieving a humidifying effect on the airflow.

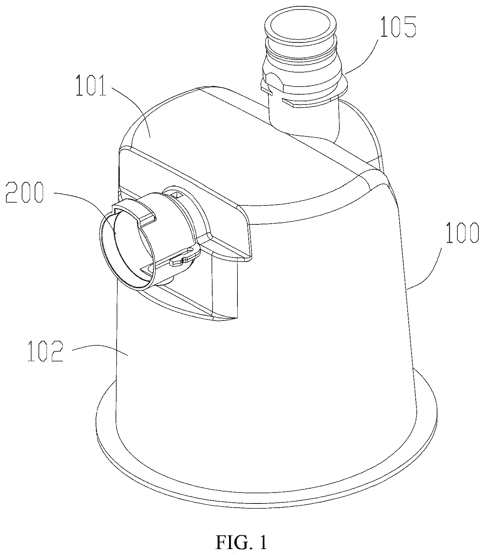

Moreover, as shown in FIG. 1, the humidification tank includes the exhaust pipe 105 connected to the hanging wall 101 of the tank body 100 and the baffle 104 is located between the flow channel and an inlet end of the exhaust pipe105. The baffle 104 may block the airflow from the flow channel from directly reaching the inlet end of the exhaust pipe 105, so that a part of the airflow flows reversely to other positions, and passes over from a position near the bottom portion to the water at the bottom portion of the tank body 100. The exhaust pipe 105 may be integrally connected to the hanging wall 101 or may be detachably connected to the hanging wall 101.

Moreover, one side edge of an inlet of the exhaust pipe 105 far away from the baffle 104 is connected to a flow guide plate 106, the inlet of the exhaust pipe 105 is located between the baffle 104 and the flow guide plate 106, and a lower end of the flow guide plate 106 is inclined towards the baffle104. As shown in FIG. 4, the inlet end of the exhaust pipe 105 is partially inserted into the cavity, the flow guide plate 106 may be connected to a partial edge of the exhaust pipe 105, the lower end of the flow guide plate 106 is inclined towards the baffle 104, and a channel is formed between the flow guide plate 106 and the baffle 104 to prevent the airflow from directly entering the exhaust pipe 105 in a vertical upward direction, thus prolonging a path before the airflow enters the exhaust pipe 105 to a certain extent.

The baffle 104 is recessed towards the exhaust pipe 105. Both sides of the baffle 104 are inclined towards the exhaust pipe 105 relative to a middle portion to form a middle recess, and the baffle 104 and the flow guide plate 106 form the channel to allow the airflow to rise into the exhaust pipe 105. Moreover, as shown in FIG. 6, the baffle 104 may also be formed in a flat plate form.

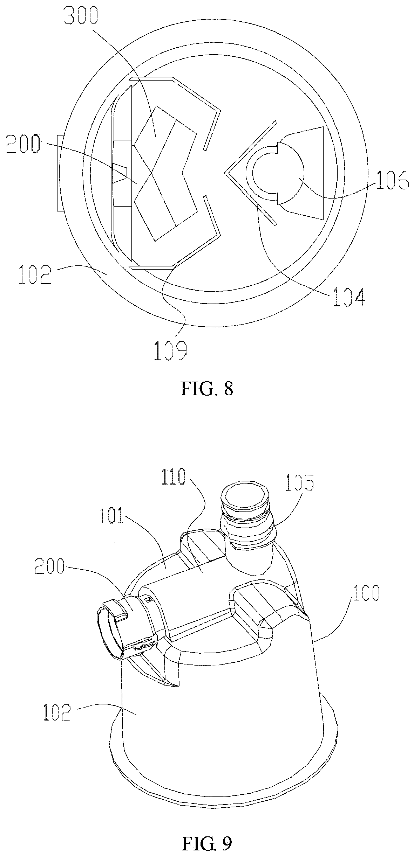

A surface of the baffle 104 facing the inlet end of the exhaust pipe 105 is an arc-shaped surface, and a central axis of the arc-shaped surface extends vertically. Alternatively, the baffle 104 includes at least two flat plate portions connected in turn. As shown in FIG. 5, the baffle 104 is formed in an arc-shaped structure with a central axis extending substantially vertically and a recessed surface facing the side on which the exhaust pipe 105 is disposed. As shown in FIG. 7, the baffle 104 includes four flat plate portions connected in turn to form a concave structure in the middle and the flat plates on both sides of the baffle 104 are inclined towards the exhaust pipe 105. As shown in FIG. 8, the baffle 104 includes two angled flat plate portions. FIG. 5, FIG. 7, and FIG. 8 show different forms of the baffles 104 recessed in the middle respectively.

An outer peripheral surface of the joint pipe 200 is provided with a buckle 201, and the side wall 102 is provided with a clamping groove 107 which can be clamped and matched with the buckle 201. The buckle 201 protrudes from the outer peripheral surface of the joint pipe 200, and the clamping groove 107 is formed on a hole wall of the side wall 102 that accommodates the joint pipe 200 to pass through, and the buckle 201 may be inserted into the clamping groove 107 to achieve locking between the joint pipe 200 and the side wall 102. The buckle 201 has an inclined surface gradually raised from one end to the other end to guide the buckle 201 to be inserted into the hole of the side wall 102, and forms an end face perpendicular to a length direction of the joint pipe 200 at the other end so as to be clamped in the clamping groove 107.

Moreover, the outer circumferential surface of the joint pipe 200 is provided with a guide rib 202, and the side wall 102 is provided with a guide groove 108 which can accommodate insertion of the guide rib 202. A width of the guide rib 202 gradually increases along the length direction of the joint pipe 200, allowing the guide rib 202 to be more easily inserted into the guide groove 108 on the hole wall of the side wall 102. The cooperation of the guide rib 202 with the guide groove 108 may guide the insertion of the joint pipe 200 into the side wall 102.

In addition, the present application further provides a ventilation treatment device, wherein the ventilation treatment device includes the humidification tank described in the above solutions. The ventilation treatment device includes a ventilator and a humidification tank. The ventilator directs an airflow into the humidification tank through an air guide pipe to humidify the airflow.

### Embodiment 2

The present application provides a humidification tank, wherein the humidification tank includes a tank body 100 provided with a cavity and a joint pipe 200 inserted into the cavity, and a part of the joint pipe 200 located in the cavity is provided with a plurality of air holes 203 at intervals along a circumferential direction.

The tank body 100 is provided with the cavity for containing liquid, such as containing water. The joint pipe 200 is inserted into the cavity of the tank body 100, and one end of the joint pipe located outside the cavity may be connected to an air guide pipe, so that gas can be input into the cavity. The air holes 203 are distributed at intervals along the circumferential direction, so that gas in the joint pipe 200 can be discharged into the cavity of the tank body 100 through the air holes 203 in different angle radial directions, the gas from the joint pipe 200 can reach more positions in the cavity in different directions, so that a situation that positions which cannot be blown by the gas exist in the cavity can be avoided, airflow dead zones are avoided, gas flowability in the cavity is improved, and a gas flowing path is prolonged, so that a humidifying effect of the gas is improved.

The tank body 100 is connected to an exhaust pipe 105, the exhaust pipe 105 can exhaust the humidified gas, and the exhaust pipe 105 may be connected to the air guide pipe to output the gas to a target position.

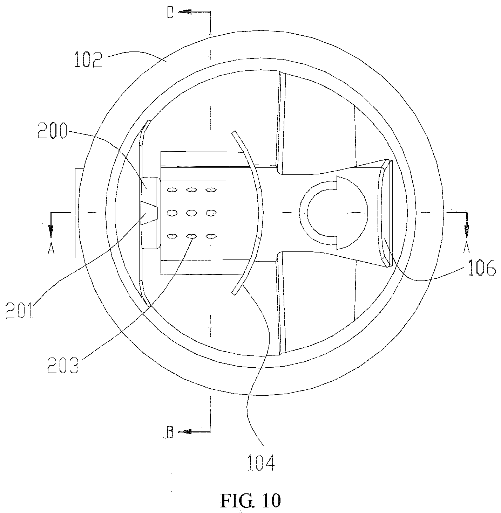

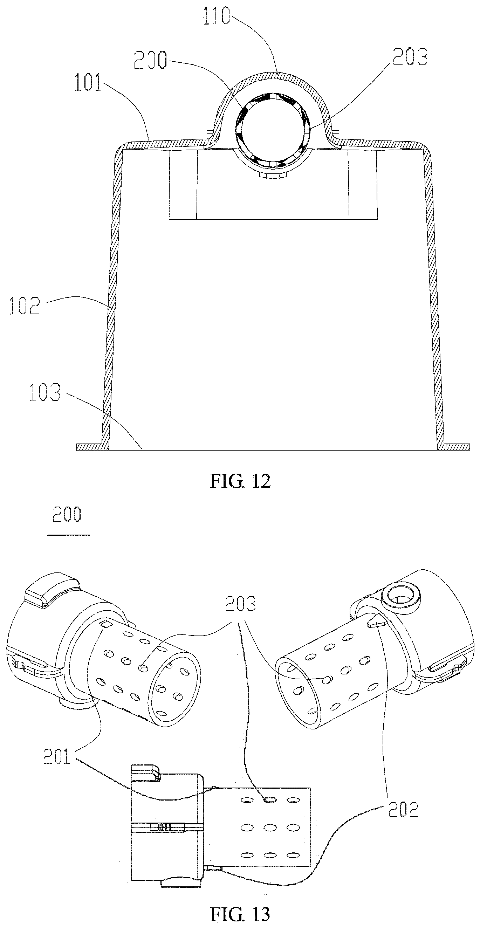

In addition, the plurality of air holes 203 are distributed in an array along circumferential and axial directions of the joint pipe 200. As shown in FIG. 10 and FIG. 13, the air holes 203 are arranged at intervals in a length direction of the joint pipe 200 on the part where the joint pipe 200 is inserted into the cavity, and as described above, the air holes are arranged at intervals in the circumferential direction of the joint pipe 200 to form an air hole array, so that the airflow can be discharged in more directions through the air holes 203, such that the airflow reaches more positions in the cavity. The air holes 203 may be arranged regularly or irregularly.

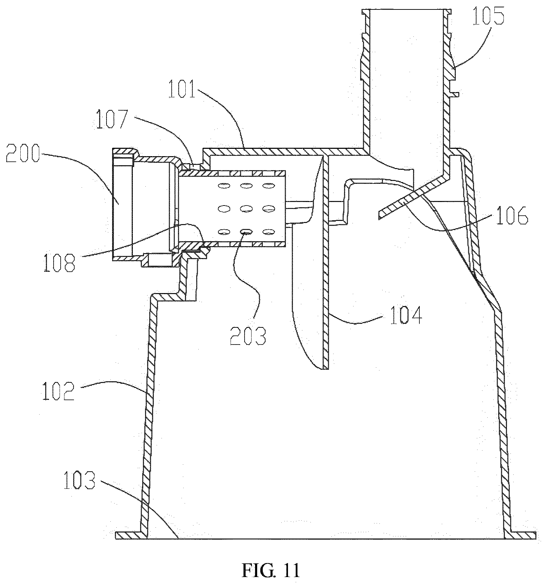

Moreover, the tank body 100 includes a hanging wall 101 with the joint pipe 200 extending horizontally and spaced from the hanging wall 101. With reference to FIG. 11 and FIG. 12, the joint pipe 200 is inserted into the cavity from a side portion of the tank body 100 (i.e., a side wall 102) and extends substantially horizontally (when the tank body 100 is horizontally placed), with the hanging wall 101 of the tank body 100 being spaced from the part where the joint pipe 200 is inserted into the cavity to prevent the hanging wall 101 from blocking a part of the air holes 203 from discharging gas.

Further, the hanging wall 101 includes an arc-shaped portion 110 covering the joint pipe 200. With reference to FIG. 6 and FIG. 12, the hanging wall 101 includes a part spaced from a partial periphery of the joint pipe 200, i.e. the arc-shaped portion 110, and the gas discharged towards the air hole 203 of the arc-shaped portion 110 flows to both sides under the guidance of the arc-shaped portion 110, which enables the airflow to flow near an inner surface of the hanging wall 101, so that the airflow reaches more positions.

In addition, the cavity is provided with a baffle 104 spaced from an outlet end of the joint pipe 200. With reference to FIG. 10 and FIG. 11, the outlet end of the joint pipe 200 faces the baffle 104, and an airflow discharged from the outlet end of the joint pipe 200 may blow to the surface of the baffle 104, and the baffle 104 may change a direction of the airflow and guide the airflow to other positions, thus prolonging a gas flowing path and improving a humidifying effect.

As shown in FIG. 9, the humidification tank includes the exhaust pipe 105 connected to the hanging wall 101 and the baffle 104 is located between the joint pipe 200 and the exhaust pipe 105. Referring to FIG. 11, the outlet end of the joint pipe 200 faces an inlet end of the exhaust pipe105, and the baffle 104 may prevent the airflow from the outlet end of the joint pipe 200 from reaching the inlet end of the exhaust pipe105 directly, and the baffle 104 changes the direction of the airflow from the outlet end of the joint pipe 200, so that the airflow passes through other positions in the cavity before entering the inlet end of the exhaust pipe105. The exhaust pipe 105 may be integrally connected to the hanging wall 101 or may be detachably connected to the hanging wall 101.

The baffle 104 may be connected to the hanging wall 101, and a lower end of the baffle may be spaced from a bottom wall 103 of the tank body 100 to allow the airflow to pass over a lower side of the baffle 104, so that the airflow may be closer to the bottom wall 103 of the tank body 100, i.e. closer to the water at a bottom portion of the tank body 100, thereby achieving a humidifying effect on the airflow. In other embodiment, two sides or other one side of the baffle 104 may be connected to the side wall 102 of the tank body 100.

The baffle 104 is recessed towards the joint pipe 200. A surface of the baffle 104 facing the joint pipe 200 is recessed, wherein an edge portion of the baffle 104 is inclined towards the joint pipe 200 relative to a middle portion to form a middle recess. After the airflow discharged from the outlet end of the joint pipe 200 reaches the middle portion, the airflow changes direction along the inclined surface and can reversely reach a rear side position of the outlet end of the joint pipe 200, thus prolonging a flowing path.

Further, the surface of the baffle 104 facing the joint pipe 200 is an arc-shaped surface, and a central axis of the arc-shaped surface extends vertically. Referring to FIG. 10, the baffle 104 is substantially an arc-shaped plate with a central axis extending vertically, and the airflow reaching the baffle 104 changes direction along the arc-shaped surface to reach the rear side position of the joint pipe 200. In other embodiments, the surface of the baffle 104 may also be a spherical recess and the baffle 104 is similar to a partially spherical shell-like structure.

Moreover, one side edge of an inlet of the exhaust pipe 105 far away from the baffle 104 is connected to a flow guide plate 106, the inlet of the exhaust pipe 105 is located between the flow guide plate 106 and the baffle 104, and a lower end of the flow guide plate 106 is inclined towards the baffle104. As shown in FIG. 11, the inlet end of the exhaust pipe 105 is partially inserted into the cavity, the flow guide plate 106 may be connected to a partial edge of the exhaust pipe 105, the lower end of the flow guide plate 106 is inclined towards the baffle 104, and a channel is formed between the flow guide plate 106 and the baffle 104 to prevent the airflow from directly entering the exhaust pipe 105 in a vertical upward direction, thus prolonging a path before the airflow enters the exhaust pipe 105 to a certain extent.

An opening of the joint pipe 200 located in the cavity is provided with a sealing wall. That is, the outlet end of the joint pipe 200 is sealed, for example, connected to one sealing wall, so as to prevent the gas from being discharged from the outlet end of the joint pipe 200, but only through the air holes 203 on the outer periphery of the joint pipe 200.

In addition, the tank body 100 includes the side wall 102, and the joint pipe 200 passes through the side wall 102 and is inserted into the cavity of the tank body 100. As described above, the joint pipe 200 is horizontally inserted into the cavity of the tank body 100. In other embodiments, the joint pipe 200 may also form a certain angle with a horizontal plane.

An outer peripheral surface of the joint pipe 200 is provided with a buckle 201, and the side wall 102 is provided with a clamping groove 107 which can be clamped and matched with the buckle 201. As shown in FIG. 13, the buckle 201 protrudes from the outer peripheral surface of the joint pipe 200, and the clamping groove 107 is formed on a hole wall of the side wall 102 that accommodates the joint pipe 200 to pass through, and the buckle 201 may be inserted into the clamping groove 107 to achieve locking between the joint pipe 200 and the side wall 102. The buckle 201 has an inclined surface gradually raised from one end to the other end to guide the buckle 201 to be inserted into the hole of the side wall 102, and forms an end face perpendicular to a length direction of the joint pipe 200 at the other end so as to be clamped in the clamping groove 107.

Moreover, the outer circumferential surface of the joint pipe 200 is provided with a guide rib 202, and the side wall 102 is provided with a guide groove 108 which can accommodate insertion of the guide rib 202. As shown in FIG. 13, a width of the guide rib 202 gradually increases along the length direction of the joint pipe 200, allowing the guide rib 202 to be more easily inserted into the guide groove 108 on the hole wall of the side wall 102. The cooperation of the guide rib 202 with the guide groove 108 may guide the insertion of the joint pipe 200 into the side wall 102.

In addition, the present application further provides a ventilation treatment device, wherein the ventilation treatment device includes the humidification tank described in the above solutions. The ventilation treatment device includes a ventilator and a humidification tank. The ventilator directs an airflow into the humidification tank through an air guide pipe to humidify the airflow.

### Embodiment 3

The present application provides a humidification tank, wherein the humidification tank includes a tank body 100 provided with a cavity, a joint pipe 200 inserted into the cavity and a baffle 104 located in the cavity and spaced from an outlet end of the joint pipe 200, and the baffle 104 is recessed towards the joint pipe 200.

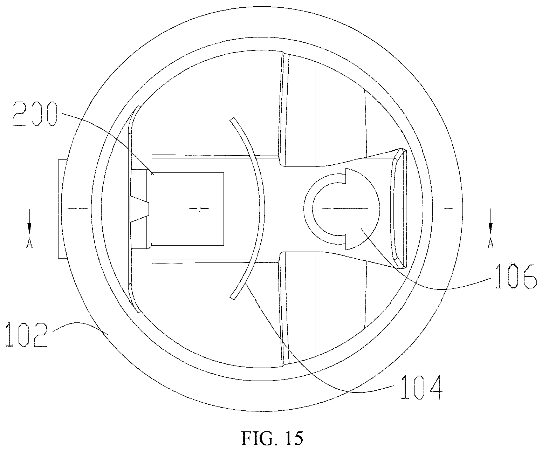

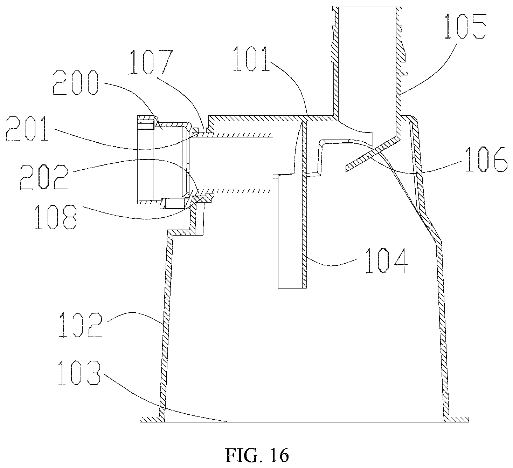

The tank body 100 is provided with the cavity for containing liquid, such as containing water. The joint pipe 200 is inserted into the cavity of the tank body 100, and one end of the joint pipe located outside the cavity may be connected to an air guide pipe, so that gas can be input into the cavity. With reference to FIG. 15 and FIG. 16, the outlet end of the joint pipe 200 faces the baffle 104, and an airflow discharged from the outlet end of the joint pipe 200 may blow to the surface of the baffle 104. A surface of the baffle 104 facing the joint pipe 200 is recessed, so that the airflow blown to the baffle 104 can flow reversely. Certainly, a flow direction of this airflow is not completely opposite to the outlet direction of the joint pipe 200. Instead, there are components in multiple directions, especially those components opposite to the outlet direction of the joint pipe 200. The baffle 104 changes the direction of the airflow, so that the airflow is distributed along edges of the baffle 104 to guide the airflow to more other positions, especially some areas behind the outlet end of the joint pipe 200, prolonging a gas flowing path and improving a humidifying effect.

The tank body 100 includes a hanging wall 101, and the baffle 104 is connected to the hanging wall 101. The baffle 104 may prevent the gas exhausted from the joint pipe 200 be collected at the hanging wall 101, and a lower end of the baffle 104 may be spaced from a bottom wall 103 of the tank body 100 to allow the airflow to pass over a lower side of the baffle 104, so that the airflow may be closer to the bottom wall 103 of the tank body 100, i.e. closer to the water at a bottom portion of the tank body 100, thereby achieving a humidifying effect on the airflow. In other embodiment, two sides or other one side of the baffle 104 may be connected to the side wall 102 of the tank body 100.

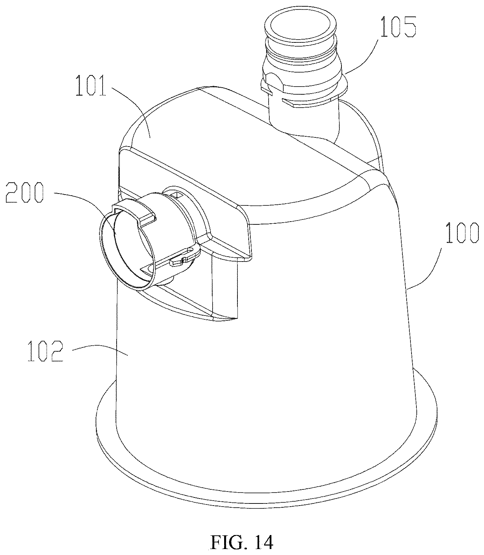

Moreover, as shown in FIG. 14, the humidification tank includes an exhaust pipe 105 connected to the hanging wall 101 and the baffle 104 is located between an inlet end of the exhaust pipe105 and the outlet end of the joint pipe 200. Referring to FIG. 16, the outlet end of the joint pipe 200 faces an inlet end of the exhaust pipe105, and the baffle 104 may prevent the airflow from the outlet end of the joint pipe 200 from reaching the inlet end of the exhaust pipe105 directly, and the baffle 104 changes the direction of the airflow from the outlet end of the joint pipe 200, so that the airflow passes through other positions in the cavity before entering the inlet end of the exhaust pipe105. The exhaust pipe 105 may be integrally connected to the hanging wall 101 or may be detachably connected to the hanging wall 101.

Moreover, one side edge of an inlet of the exhaust pipe 105 far away from the baffle 104 is connected to a flow guide plate 106, the inlet of the exhaust pipe 105 is located between the flow guide plate 106 and the baffle 104, and a lower end of the flow guide plate 106 is inclined towards the baffle104. As shown in FIG. 16, the inlet end of the exhaust pipe 105 is partially inserted into the cavity, the flow guide plate 106 may be connected to a partial edge of the exhaust pipe 105, the lower end of the flow guide plate 106 is inclined towards the baffle 104, and a channel is formed between the flow guide plate 106 and the baffle 104 to prevent the airflow from directly entering the exhaust pipe 105 in a vertical upward direction, thus prolonging a path before the airflow enters the exhaust pipe 105 to a certain extent.

A middle portion of the baffle 104 facing the joint pipe 200 is recessed with respect to portions on both lateral sides thereof. Edge portions of the two lateral sides of the baffle 104 are inclined towards the joint pipe 200 relative to the middle portion to form a middle recess. After the airflow discharged from the outlet end of the joint pipe 200 reaches the middle portion, the airflow changes direction along the inclined surface and can reversely reach rear side positions at the two sides of the outlet end of the joint pipe 200, thus prolonging a flowing path.

Further, the surface of the baffle 104 facing the joint pipe 200 is an arc-shaped surface, and a central axis of the arc-shaped surface extends vertically. Referring to FIG. 15, the baffle 104 is substantially an arc-shaped plate with a central axis extending vertically, and the airflow reaching the baffle 104 changes direction along the arc-shaped surface to reach the rear side position of the joint pipe 200. In other embodiments, the surface of the baffle 104 may also be a spherical recess and the baffle 104 is similar to a partially spherical shell-like structure.

In addition, the tank body 100 includes the side wall 102, and the joint pipe 200 passes through the side wall 102 and is inserted into the cavity of the tank body 100. As described above, the joint pipe 200 is horizontally inserted into the cavity of the tank body 100. In other embodiments, the joint pipe 200 may also form a certain angle with a horizontal plane.

An outer peripheral surface of the joint pipe 200 is provided with a buckle 201, and the side wall 102 is provided with a clamping groove 107 which can be clamped and matched with the buckle 201. The buckle 201 protrudes from the outer peripheral surface of the joint pipe 200, and the clamping groove 107 is formed on a hole wall of the side wall 102 that accommodates the joint pipe 200 to pass through, and the buckle 201 may be inserted into the clamping groove 107 to achieve locking between the joint pipe 200 and the side wall 102.The buckle 201 has an inclined surface gradually raised from one end to the other end to guide the buckle 201 to be inserted into the hole of the side wall 102, and forms an end face perpendicular to a length direction of the joint pipe 200 at the other end so as to be clamped in the clamping groove 107.

Moreover, the outer circumferential surface of the joint pipe 200 is provided with a guide rib 202, and the side wall 102 is provided with a guide groove 108 which can accommodate insertion of the guide rib 202. A width of the guide rib 202 gradually increases along the length direction of the joint pipe 200, allowing the guide rib 202 to be more easily inserted into the guide groove 108 on the hole wall of the side wall 102. The cooperation of the guide rib 202 with the guide groove 108 may guide the insertion of the joint pipe 200 into the side wall 102.

In addition, the present application further provides a ventilation treatment device, wherein the ventilation treatment device includes the humidification tank described in the above solutions. The ventilation treatment device includes a ventilator and a humidification tank. The ventilator directs an airflow into the humidification tank through an air guide pipe to humidify the airflow.

### Embodiment 4

The present application provides a humidifying tank, wherein the humidifying tank includes a tank body 100 provided with a cavity, a joint pipe 200 inserted into the cavity, and a baffle 104. The baffle 104 extends from an A side to a B side in a lateral direction of an outlet end of the joint pipe 200, and the baffle 104 deflects away from the joint pipe 200.

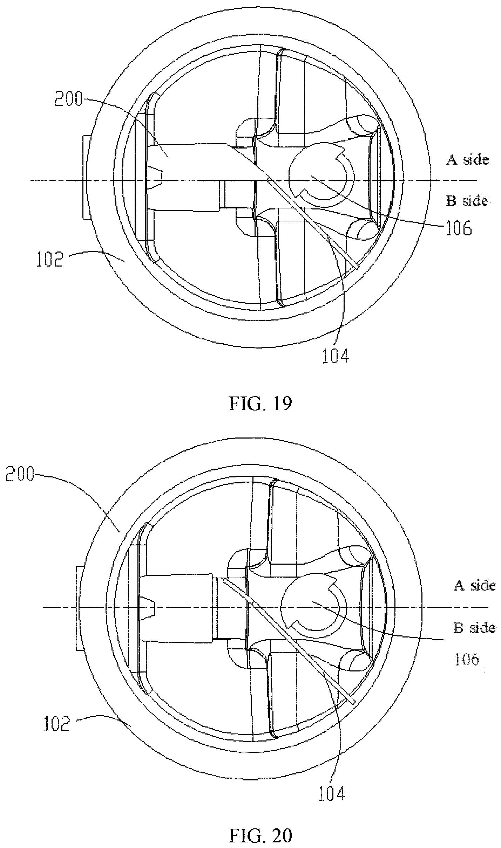

In this embodiment, referring to FIG. 19 and FIG. 20, the cavity is divided into two lateral parts with a central axis of the joint pipe 200 as the boundary, one lateral side of the central axis is the A side, the other lateral side is the B side, the A side may be a left side or a right side, and correspondingly, the B side may be the right side or the left side.

The tank body 100 is provided with the cavity for containing liquid, such as containing water. The joint pipe 200 is inserted into the cavity of the tank body 100, and one end of the joint pipe located outside the cavity may be connected to an air guide pipe, so that gas can be input into the cavity. With reference to FIG. 19 and FIG. 20, the baffle 104 extends from one lateral side (A side) of the joint pipe 200 to the other lateral side (B side), and a portion of the baffle 104 corresponding to (towards or aligned) the B side of the joint pipe 200 is inclined away from the joint pipe 200 relative to a portion of the baffle 104 corresponding to (towards or aligned) the A side of the joint pipe 200; or, a distance between the portion of the baffle 104 corresponding to the B side of the joint pipe 200 and the joint pipe 200 is greater than a distance between the portion of the baffle 104 corresponding to the A side of the joint pipe 200 and the joint pipe 200. This causes the gas discharged from the joint pipe 200 to be discharged from the B side, and flows along a surface of the baffle 104 towards an edge of the cavity of the tank body 100, and further flows along the edge of the cavity, so that the airflow can form a substantially spiral path, so as to flow to more positions in the cavity, avoid the existence of positions in the cavity where the gas cannot be blown, avoid airflow dead zones, improve a gas liquidity inside the cavity, prolong a gas flowing path, and thus improves a humidifying effect of the gas.

Further, a side edge of the baffle 104 on the B side is connected to a side wall 102 of the tank body 100. With reference to FIG. 19, FIG. 20, FIG. 23, and FIG. 27, the side edge of the baffle 104 corresponding to the B side of the joint pipe 200 is connected to the side wall 102, and the airflow flows along the baffle 104 towards the side wall 102. When reaching a turning direction of the side wall 102, the airflow flows along the side wall 102, so that the airflow can pass through the edge of the cavity and reach a side of the baffle 104 facing away from the joint pipe 200. A side edge of the baffle 104 located on the A side may be connected to the A side of the joint pipe 200 to block the airflow in the joint pipe 200 from being discharged from the A side.

As shown in FIG.19, a portion of the joint pipe 200 located on the A side is connected to the baffle 104, and a portion of the joint pipe 200 located on the B side forms an opening to allow the airflow to flow out of the opening and flow along the surface of the baffle 104 toward the edge of the cavity.

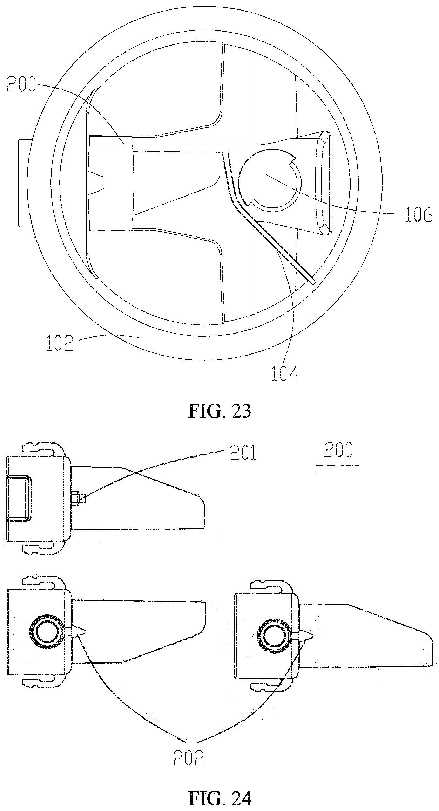

The opening is formed on the B side of the outlet end of the joint pipe 200. As shown in FIG. 23 to FIG. 25, the outlet end of the joint pipe 200 is not flush, but is beveled, forming the opening on the B side, so that the airflow is deflected from the opening towards the B side.

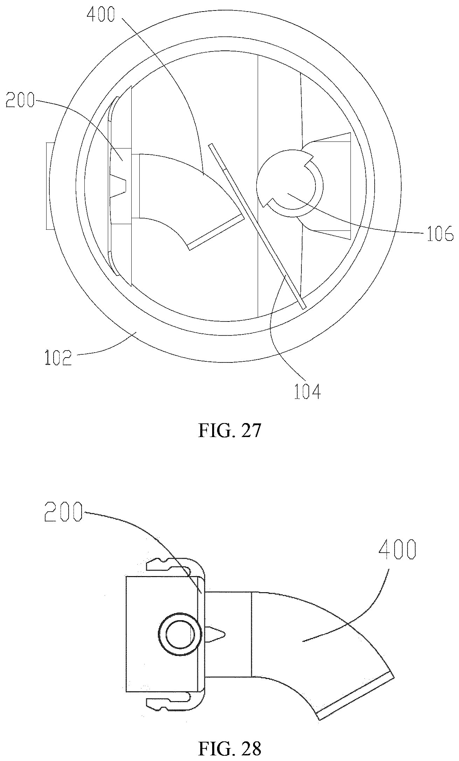

According to another embodiment of the present solution, the outlet end of the joint pipe 200 is connected to an elbow 400 bent from the A side to the B side. As shown in FIG. 27 and FIG. 28, the joint pipe 200 is connected to the elbow 400, and the elbow 400 bends to the B side, which can also deflect the airflow to the B side and blow the airflow along the baffle 104 to the edge of the cavity, that is, to the side wall 102.

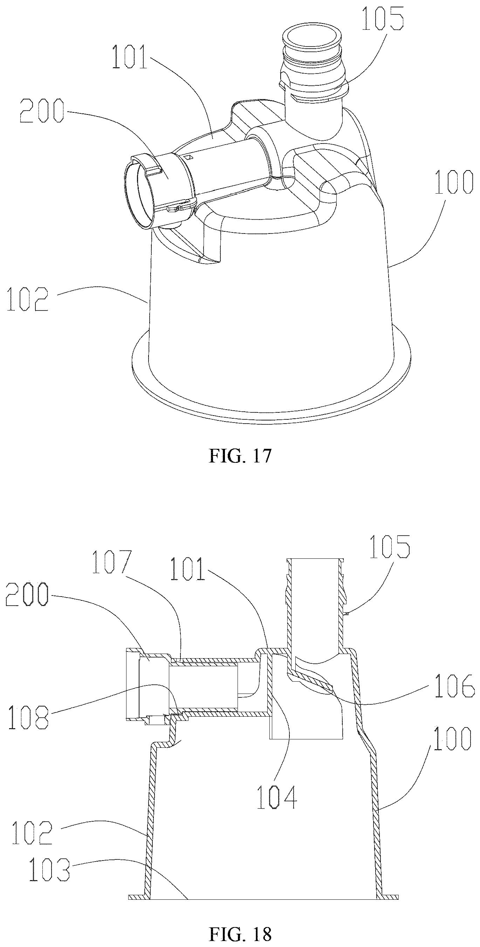

A hanging wall 101 of the tank body 100 is connected to an exhaust pipe 105, and the baffle 104 is located between the joint pipe 200 and an inlet end of the exhaust pipe 105. Referring to FIG. 17, FIG. 22 and FIG. 26, a top portion of the tank body 100 is provided with the hanging wall 101. As shown in FIG. 18, the outlet end of the joint pipe 200 faces the inlet end of the exhaust pipe 105, and the baffle 104 can block the airflow from the outlet end of the joint pipe 200 from directly reaching the inlet end of the exhaust pipe 105. The baffle 104 changes a direction of the airflow from the outlet end of the joint pipe 200, so that the airflow passes through other positions in the cavity before entering the inlet end of the exhaust pipe 105. The exhaust pipe 105 may be integrally connected to the hanging wall 101 or may be detachably connected to the hanging wall 101.

In addition, the baffle 104 is connected to the hanging wall 101 of the tank body 100 and spaced from a bottom wall 103 of the tank body 100. The baffle 104 may prevent the gas exhausted from the joint pipe 200 be collected at the hanging wall 101, and a lower end of the baffle 104 may be spaced from a bottom wall 103 of the tank body 100, so that the airflow may also pass over a lower side of the baffle 104, so that the airflow may be closer to the bottom wall 103 of the tank body 100, i.e. closer to the water at a bottom portion of the tank body 100, thereby achieving a humidifying effect on the airflow.

Moreover, one side edge of an inlet of the exhaust pipe 105close to the baffle 104 is connected to a flow guide plate 106, the flow guide plate 106 is located between the baffle 104 and the inlet end of the exhaust pipe105, and a lower end of the flow guide plate 106 is inclined away from the baffle104. As shown in FIG. 18, the inlet end of the exhaust pipe 105 is partially inserted into the cavity, the flow guide plate106 is connected to a partial edge of the exhaust pipe 105, the lower end of the flow guide plate 106 is inclined towards the baffle 104, and a channel smaller than an inner diameter of the exhaust pipe 105 is formed between the side wall 102 and the baffle 104, so that the airflow flowing along the side wall 102 can enter the exhaust pipe 105 upwards, and the airflow can be prevented from directly entering the exhaust pipe 105 in a vertical upward direction, thus prolonging a path before the airflow enters the exhaust pipe 105 to a certain extent.

The joint pipe 200 passes through the side wall 102 of the tank body 100 and extends horizontally. As described above, the joint pipe 200 is horizontally inserted into the cavity of the tank body 100. In other embodiments, the joint pipe 200 may also form a certain angle with a horizontal plane.

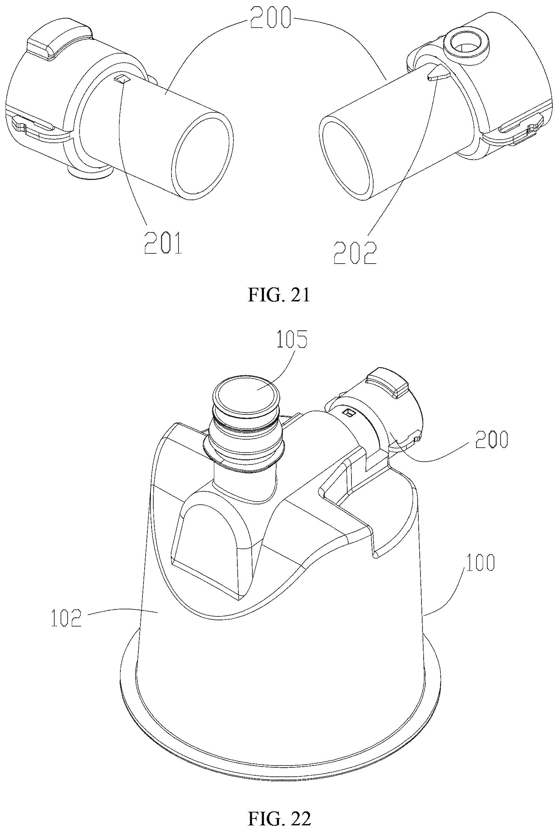

Moreover, the periphery of the joint pipe 200 is provided with a guide rib 202, and the side wall 102 is provided with a guide groove 108 which can accommodate insertion of the guide rib 202. As shown in FIG. 21 and FIG. 24, a width of the guide rib 202 gradually increases along the length direction of the joint pipe 200, allowing the guide rib 202 to be more easily inserted into the guide groove 108 on the hole wall of the side wall 102. The cooperation of the guide rib 202 with the guide groove 108 may guide the insertion of the joint pipe 200 into the side wall 102.

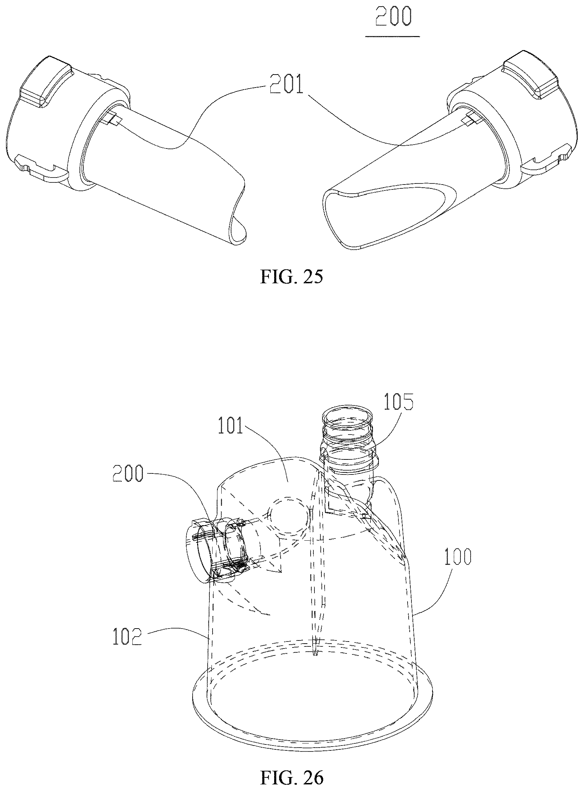

Moreover, an outer peripheral surface of the joint pipe 200 is provided with a buckle 201, and the side wall 102 is provided with a clamping groove 107 which can be clamped and matched with the buckle 201. As shown in FIG. 21 and FIG. 25, the buckle 201 protrudes from the outer peripheral surface of the joint pipe 200, and the clamping groove 107 is formed on a hole wall of the side wall 102 that accommodates the joint pipe 200 to pass through, and the buckle 201 may be inserted into the clamping groove 107 to achieve locking between the joint pipe 200 and the side wall 102. The buckle 201 has an inclined surface gradually raised from one end to the other end to guide the buckle 201 to be inserted into the hole of the side wall 102, and forms an end face perpendicular to a length direction of the joint pipe 200 at the other end so as to be clamped in the clamping groove 107.

In addition, the present application further provides a ventilation treatment device, wherein the ventilation treatment device includes the humidification tank described in the above solutions. The ventilation treatment device includes a ventilator and a humidification tank. The ventilator directs an airflow into the humidification tank through an air guide pipe to humidify the airflow. The invention is defined by the claims which follow.

## Claims

1. A humidification tank comprises a tank body (100) provided with a cavity, a joint pipe (200) inserted into the cavity, and at least two branch pipes (300) communicated with an outlet end of the joint pipe (200),
**characterized in that**
the cavity is provided with two deflection plates (109) each facing one of outlet ends of the branch pipes (300), and the deflection plates (109) are configured to change a direction of gas flowing out of the outlet ends of the branch pipes (300),
wherein ends of the two deflection plates (109) close to each other are spaced to form a flow channel.

2. The humidification tank according to claim 1, wherein the outlet end of the joint pipe (200) is connected to the two branch pipes (300) which extend horizontally, and the two branch pipes (300) and the joint pipe (200) form a Y-shaped three-way pipe.

3. The humidification tank according to claim 1, wherein one ends of the two deflection plates (109) far away from each other are both connected to a side wall (102) of the tank body (100).

4. The humidification tank according to claim 1 or 3, wherein the deflection plate (109) is connected to a hanging wall (101) of the tank body (100), and the deflection plate (109) is spaced from a bottom wall (103) of the tank body (100).

5. The humidification tank according to any one of claims 1 to 4, wherein the deflection plate (109) is recessed towards the outlet end of the branch pipe (300).

6. The humidification tank according to any one of claims 1 to 5, wherein the cavity is provided with a baffle (104) facing the flow channel, and the baffle (104) is configured to change the direction of the gas flowing out of the outlet of the branch pipe (300).

7. The humidification tank according to claim 6, wherein two lateral sides of the baffle (104) are spaced from the side wall (102) of the tank body (100).

8. The humidification tank according to claim 7, wherein the baffle (104) is connected to the hanging wall (101) of the tank body (100), and the baffle (104) is spaced from the bottom wall (103) of the tank body (100).

9. The humidification tank according to claim 7 or claim 8, wherein the humidification tank comprises an exhaust pipe (105) connected to the hanging wall (101) of the tank body (100), and the baffle (104) is located between the flow channel and an inlet end of the exhaust pipe (105).

10. The humidification tank according to claim 9, wherein one side edge of an inlet of the exhaust pipe (105) far away from the baffle (104) is connected to a flow guide plate (106), and a lower end of the flow guide plate (106) is inclined towards the baffle (104).

11. The humidification tank according to claim 9 or 10, wherein the baffle (104) is recessed towards the inlet end of the exhaust pipe (105).

12. A ventilation treatment device, **characterized in that** the ventilation treatment device comprises the humidification tank according to any one of claims 1 to 11.

## Patentansprüche

1. Befeuchtungstank, der einen Tank-Körper (100), der mit einem Hohlraum, einem in den Hohlraum eingeführten Verbindungsrohr (200) sowie wenigstens zwei Abzweigohren (300) versehen ist, die mit einem Auslassende des Verbindungsrohrs (200) in Verbindung stehen,
**dadurch gekennzeichnet, dass**
der Hohlraum mit zwei Ablenkplatten (109) versehen ist, die jeweils einem von Auslassenden der Abzweigrohre (300) zugewandt sind, und die Ablenkplatten (109) zum Ändern einer Richtung von Gas ausgeführt sind, das an den Auslassenden der Abzweigrohre (300) ausströmt, wobei
Enden der zwei Ablenkplatten (109), die einander nahe sind, so beabstandet sind, dass sie einen Strömungskanal bilden.

2. Befeuchtungstank nach Anspruch 1, wobei das Auslassende des Verbindungsrohrs (200) mit den zwei Abzweigrohren (300) verbunden ist, die sich horizontal erstrecken, und die zwei Abzweigohre (300) und das Verbindungsrohr (200) ein Y-förmiges Gabelrohr bilden.

3. Befeuchtungstank nach Anspruch 1, wobei weit voneinander entfernte eine Enden der zwei Ablenkplatten (109) beide mit einer Seitenwand (102) des Tank-Körpers (100) verbunden sind.

4. Befeuchtungstank nach Anspruch 1 oder 3, wobei die Ablenkplatte (109) mit einer Decke (101) des Tank-Körpers (101) verbunden ist und die Ablenkplatte (109) von einer Bodenwand (103) des Tank-Körpers (100) beabstandet ist.

5. Befeuchtungstank nach einem der Ansprüche 1 bis 4, wobei die Ablenkplatte (109) in Richtung des Auslassendes des Abzweigrohrs (300) vertieft ist.

6. Befeuchtungstank nach einem der Ansprüche 1 bis 5, wobei der Hohlraum mit einer den Strömungskanal zugewandten Umleitplatte (104) versehen ist und die Umleitplatte (104) so ausgeführt ist, dass sie die Richtung des Gases ändert, das über den Auslass des Abzweigrohrs (300) ausströmt.

7. Befeuchtungstank nach Anspruch 6, wobei zwei Längsseiten der Umleitplatte (104) von der Seitenwand (102) des Tank-Körpers (100) beabstandet sind.

8. Befeuchtungstank nach Anspruch 7, wobei die Umleitplatte (104) mit der Decke (101) des Tank-Körpers (101) verbunden ist und die Umleitplatte (104) von der Bodenwand (103) des Tank-Körpers (100) beabstandet ist.

9. Befeuchtungstank nach Anspruch 7 oder Anspruch 8, wobei der Befeuchtungstank ein Ableitrohr (105) umfasst, das mit der Decke (101) des Tank-Körpers (100) verbunden ist, und die Umleitplatte (104) sich zwischen dem Strömungskanal und einem Einlassende des Ableitrohrs (105) befindet.

10. Befeuchtungstank nach Anspruch 9, wobei ein von der Umleitplatte (104) entfernter Seitenrand eines Einlasses des Ableitrohrs (104) mit einer Strömungs-Leitplatte (106) verbunden ist und ein unteres Ende der Strömungs-Leitplatte (106) in Richtung der Umleitplatte (104) geneigt ist.

11. Befeuchtungstank nach Anspruch 9 oder 10, wobei die Ablenkplatte (104) in Richtung des Einlassendes des Ableitrohrs (105) vertieft ist.

12. Beatmungsbehandlungs-Vorrichtung, **dadurch gekennzeichnet, dass** die Beatmungs-behandlungs-Vorrichtung den Befeuchtungstank nach einem der Ansprüche 1 bis 11 umfasst.

## Revendications

1. Réservoir d'humidification comprenant un corps de réservoir (100) pourvu d'une cavité, un tuyau de raccordement (200) inséré dans la cavité, et au moins deux tuyaux de dérivation (300) communiquant avec une extrémité de sortie du tuyau de raccordement (200),
**caractérisé en ce que**
la cavité est pourvue de deux plaques de déflexion (109) faisant face chacune à l'une des extrémités de sortie des tuyaux de dérivation (300), et les plaques de déflexion (109) sont configurées pour changer la direction du gaz s'écoulant hors des extrémités de sortie des tuyaux de dérivation (300),
dans lequel les extrémités des deux plaques de déflexion (109) proches l'une de l'autre sont espacées pour former un canal d'écoulement.

2. Réservoir d'humidification selon la revendication 1, dans lequel l'extrémité de sortie du tuyau de raccordement (200) est connectée aux deux tuyaux de dérivation (300) qui s'étendent horizontalement, et les deux tuyaux de dérivation (300) et le tuyau de raccordement (200) forment un tuyau à trois voies en forme de Y.

3. Réservoir d'humidification selon la revendication 1, dans lequel les extrémités des deux plaques de déflexion (109) éloignées l'une de l'autre sont toutes deux connectées à une paroi latérale (102) du corps de réservoir (100).

4. Réservoir d'humidification selon la revendication 1 ou 3, dans lequel la plaque de déflexion (109) est connectée à une paroi suspendue (101) du corps de réservoir (100), et la plaque de déflexion (109) est espacée d'une paroi de fond (103) du corps de réservoir (100).

5. Réservoir d'humidification selon l'une quelconque des revendications 1 à 4, dans lequel la plaque de déflexion (109) est renfoncée vers l'extrémité de sortie du tuyau de dérivation (300).

6. Réservoir d'humidification selon l'une quelconque des revendications 1 à 5, dans lequel la cavité est pourvue d'un déflecteur (104) qui fait face au canal d'écoulement, et le déflecteur (104) est configuré pour changer la direction du gaz s'écoulant hors de la sortie du tuyau de dérivation (300).

7. Réservoir d'humidification selon la revendication 6, dans lequel deux côtés latéraux du déflecteur (104) sont espacés de la paroi latérale (102) du corps de réservoir (100).

8. Réservoir d'humidification selon la revendication 7, dans lequel le déflecteur (104) est connecté à la paroi suspendue (101) du corps de réservoir (100), et le déflecteur (104) est espacé de la paroi de fond (103) du corps de réservoir (100).

9. Réservoir d'humidification selon la revendication 7 ou 8, dans lequel le réservoir d'humidification comprend un tuyau d'échappement (105) connecté à la paroi suspendue (101) du corps de réservoir (100), et le déflecteur (104) est situé entre le canal d'écoulement et une extrémité d'entrée du tuyau d'échappement (105).

10. Réservoir d'humidification selon la revendication 9, dans lequel un bord latéral d'une entrée du tuyau d'échappement (105) éloigné du déflecteur (104) est connecté à une plaque de guidage d'écoulement (106), et une extrémité inférieure de la plaque de guidage d'écoulement (106) est inclinée vers le déflecteur (104).

11. Réservoir d'humidification selon la revendication 9 ou 10, dans lequel le déflecteur (104) est renfoncé vers l'extrémité d'entrée du tuyau d'échappement (105).

12. Dispositif de traitement par ventilation, **caractérisé en ce que** le dispositif de traitement par ventilation comprend le réservoir d'humidification selon l'une quelconque des revendications 1 à 11.
